# EUROPEAN PATENT APPLICATION

(11) **EP 1 514 557 A1**
(43) Date of publication of application: **16.03.2005**
(21) Application number: 03733493.5
(22) Date of filing: 18.06.2003
(51) Int. Cl.: A61K 38/17, A61K 31/7088, A61K 39/395, A61K 45/00, A61K 48/00, A61P 19/00, A61P 19/02, A61P 29/00, A61P 43/00

(54) **PREVENTIVES/REMEDIES FOR BONE AND JOINT DISEASES**

(30) Priority: 19.06.2002 JP 2002178715
(71) Applicant: Takeda Chemical Industries, Ltd., Osaka-shi, Osaka 541-0045 (JP)
(72) Inventor: WATANABE, Tatsuya, Tsukuba-shi, Ibaraki 305-0821 (JP); INAZUKA, Masakazu, Tsukuba-shi, Ibaraki 305-0035 (JP)
(74) Representative: Lewin, John Harvey
(86) International application number: PCT/JP2003/007741
(87) International publication number: WO 2004/000345

(57) **Abstract**

A compound or its salt that inhibits the activity of a protein having an amino acid sequence which is the same or substantially the same amino acid sequence represented by SEQ ID NO: 1, an antibody to the above protein, an antisense nucleotide having a base sequence which is complementary or substantially complementary to the base sequence of a DNA encoding the above protein or its peptide fragment, etc. are usable as prophylactic/therapeutic agents for bone and joint diseases.

## Description

### TECHNICAL FIELD

The present invention relates to prophylactic/therapeutic agents and diagnostic agents s for bone and joint diseases.

### BACKGROUND ART

A joint refers to a part which connects bone to bone and allows for a movement. The bones at this part are covered by articular cartilage to make the movement smooth. In a joint, synovial fluid produced by the synovial membrane works together with the articular cartilage to reduce friction and function as a lubricant, which enables a smooth movement. Osteoarthritis is a joint disorder accompanied by chronic arthritis and is a disease to cause the breakdown of cartilage and proliferative changes in bone and cartilage, due to degenerative alterations in cartilage. Also, joint deformity leading to, e.g., osteophyte formation at the ends of bones, etc. is observed. Osteoarthritis increases with age and at least 80% of people over 60 years old reportedly show symptoms of osteoarthritis at the knees, ancons, hip joints or acantha. Treatment of osteoarthritis is centered on a symptomatic treatment to relieve pain, for which a non-steroidal anti-inflammatory analgesic, hyaluronic acid or steroid drugs for direct injection into joints, etc. are used. Surgeries are performed, including arthroscopic surgery when osteoarthritis has progressed, osteotomy or artificial joint replacement for knees or hip joints when pain is strong and deformity is severe. However, it is considered that in view of the lifespan of artificial joints, it is preferable to avoid the surgery until 55 years old. It has thus been desired to develop treatment for preventing degenerative changes in joints. It ha also been desired to develop a simpler diagnosis, since osteoarthritis is only diagnosed using equipments for X-ray radiography, etc.

To exhaustively analyze gene expression, a microarray with immobilized cDNAs or oligonucleotides was developed, the technology of detecting changes in disease-specific gene expression has come into wide use, and its usefulness has been established. For example, the GeneChip system from Affymetrix Corp. is going to be in wide use.

It has been attempted to detect genes or proteins specifically expressed in joints of patients with osteoarthritis and make the discovery of drugs or diagnostic agents for osteoarthritis. For example, it is known that the expression of matrix metalloproteases (MMP) such as MMP-13, etc. increases in joints of osteoarthritis, and MMP inhibitors are considered as possible drugs for preventing joint destruction.

KIAA1077 is a human homologous gene of Qsurf1, which was identified to be a gene induced by Shh signaling in the process of developing into a quail embryo (Science, 289, 265-270, 2000). From the amino acid sequence KIAA1077 was inferred to be a sulfatase (sulfate ester hydrolyzing enzyme) and was shown to be involved in transduction of Wnt signaling, based on the studies of transfected cells (Science, 289, 265-270, 2000). However, there is no report how KIAA1077 is associated with osteoarthritis.

Safe and excellent prophylactic/therapeutic agents for bone and joint diseases have been desired.

### DIACLOSURE OF INVENTION

The present inventors have made extensive studies to solve the foregoing problems and as a result, found a gene markedly increasingly expressed in osteoarthritis cartilage. Based on this finding, the inventors have made further studies and come to accomplish the present invention.

That is, the present invention provides the following features, and so on.
(1) A prophylactic/therapeutic agent for bone and joint diseases, comprising a compound or its salt that inhibits the activity of a protein having an amino acid sequence which is the same or substantially the same as the amino acid sequence represented by SEQ ID NO: 1, its partial peptide, or a salt thereof.
(2) A prophylactic/therapeutic agent for bone and joint diseases, comprising a compound or its salt that inhibits the expression of a gene for a protein having an amino acid sequence which is the same or substantially the same as the amino acid sequence represented by SEQ ID NO: 1, its partial peptide, or a salt thereof.
(3) A prophylactic/therapeutic agent for bone and joint diseases, comprising an antisense nucleotide having a base sequence which is complementary or substantially complementary to the base sequence of a polynucleotide encoding a protein having an amino acid sequence which is the same or substantially the same as the amino acid sequence represented by SEQ ID NO: 1, or its partial peptide, or a part of the base sequence.
(4) A prophylactic/therapeutic agent for bone and joint diseases, comprising an antibody to a protein having an amino acid sequence which is the same or substantially the same as the amino acid sequence represented by SEQ ID NO: 1, its partial peptide, or a salt thereof.
(5) The prophylactic/therapeutic agent according to (1), (2), (3) or (4), wherein the bone and joint diseases are chondrodysplasia, bone dysplasia, osteoporosis, osteoarthritis, chronic articular rheumatism, arthritis, synovitis, metabolic arthropathy or sports-related joint disorders.
(6) A diagnostic agent for bone and joint diseases, comprising an antibody to a protein having an amino acid sequence which is the same or substantially the same as the amino acid sequence represented by SEQ ID NO: 1, its partial peptide, or a salt thereof.
(7) A diagnostic agent for bone and joint diseases, comprising a polynucleotide encoding a protein having an amino acid sequence which is the same or substantially the same as the amino acid sequence represented by SEQ ID NO: 1, or its partial peptide.
(8) The diagnostic agent according to (6) or (7), wherein the bone and joint diseases are chondrodysplasia, bone dysplasia, osteoporosis, osteoarthritis, chronic articular rheumatism, arthritis, synovitis, metabolic arthropathy or sports-related joint disorders.
(9) A prophylactic/therapeutic agent for bone and joint diseases, comprising a compound or its salt having an action of inhibiting a sulfatase activity.
(10) A prophylactic/therapeutic agent for bone and joint diseases, comprising a compound or its salt having an action of inhibiting the expression of a sulfatase.
(11) A method of screening a prophylactic/therapeutic agent for bone and joint diseases, which comprises using a protein having an amino acid sequence which is the same or substantially the same as the amino acid sequence represented by SEQ ID NO: 1, its partial peptide, or a salt thereof.
(11a) A method of screening a pharmaceutical compound, which comprises using a protein having an amino acid sequence which is the same or substantially the same as the amino acid sequence represented by SEQ ID NO: 1, its partial peptide, or a salt thereof.
(11b) The screening method according to (11a), wherein the compound for pharmaceutical is a compound for preventing/treating bone and joint diseases, a compound used to prevent/treat bone and joint diseases, and/or a compound having a preventing/treating effect on bone and joint diseases.
(12) A kit for screening a prophylactic/therapeutic agent for bone and joint diseases, comprising a protein having an amino acid sequence which is the same or substantially the same as the amino acid sequence represented by SEQ ID NO: 1, its partial peptide, or a salt thereof.
(12a) A kit for screening a pharmaceutical compound, comprising a protein having an amino acid sequence which is the same or substantially the same as the amino acid sequence represented by SEQ ID NO: 1, its partial peptide, or a salt thereof.
(12b) The screening kit according to (12a), wherein the compound for pharmaceutical is a compound for preventing/treating bone and joint diseases, a compound used to prevent/treat bone and joint diseases, and/or a compound having a preventing/treating effect on bone and joint diseases.
(13) A method of screening a prophylactic/therapeutic agent for bone and joint diseases, which comprises using a polynucleotide encoding a protein having an amino acid sequence which is the same or substantially the same as the amino acid sequence represented by SEQ ID NO: 1, or its partial peptide.
(13a) A method of screening a pharmaceutical compound, which comprises using a polynucleotide encoding a protein having an amino acid sequence which is the same or substantially the same as the amino acid sequence represented by SEQ ID NO: 1, or its partial peptide.
(13b) The screening method according to (13a), wherein the compound for pharmaceutical is a compound for preventing/treating bone and joint diseases, a compound used to prevent/treat bone and joint diseases, and/or a compound having a preventing/treating effect on bone and joint diseases.
(14) A kit for screening a prophylactic/therapeutic agent for bone and joint diseases, comprising a polynucleotide encoding a protein having an amino acid sequence which is the same or substantially the same as the amino acid sequence represented by SEQ ID NO: 1, or its partial peptide.
(14a) A kit for screening a pharmaceutical compound, comprising a polynucleotide encoding a protein having an amino acid sequence which is the same or substantially the same as the amino acid sequence represented by SEQ ID NO: 1, or its partial peptide.
(14b) The screening kit according to(14a), wherein the compound for pharmaceutical is a compound for preventing/treating bone and joint diseases, a compound used to prevent/treat bone and joint diseases, and/or a compound having a preventing/treating effect on bone and joint diseases.
(15) The prophylactic/therapeutic agent for bone and joint diseases, which is obtainable using the screening method according to (11) or (13), or the screening kit according to (12) or (14).
(15a) The pharmaceutical compound or its salt, which is obtainable using the screening method according to (11a) or (13a), or the screening kit according to (12a) or (14a).
(15b) The compound or its salt according to (15a), wherein the compound for pharmaceutical is a compound for preventing/treating bone and joint diseases, a compound used to prevent/treat bone and joint diseases, and/or a compound having a preventing/treating effect on bone and joint diseases.
(15c) The prophylactic/therapeutic agent for bone and joint diseases, comprising the compound or its salt according to (15a) or (15b).
(16) A method of preventing/treating bone and joint diseases, which comprises administering to a mammal an effective dose of a compound or its salt that inhibits the activity of a protein having an amino acid sequence which is the same or substantially the same as the amino acid sequence represented by SEQ ID NO: 1, its partial peptide, or a salt thereof, or an effective dose of a compound or its salt that inhibits the expression of a gene for the protein.
(17) A method of preventing/treating bone and joint diseases, which comprises inhibiting the activity of a protein having an amino acid sequence which is the same or substantially the same as the amino acid sequence represented by SEQ ID NO: 1, its partial peptide, or a salt thereof, or inhibiting the expression of a gene for the protein.
(18) Use of a compound or its salt that inhibits the activity of a protein having an amino acid sequence which is the same or substantially the same as the amino acid sequence represented by SEQ ID NO: 1, its partial peptide, or a salt thereof, or a compound or its salt that inhibits the expression of a gene for the protein, to manufacture a prophylactic/therapeutic agent for bone and joint diseases.

### BRIEF DESCRIPTION OF THE DRAWINGS

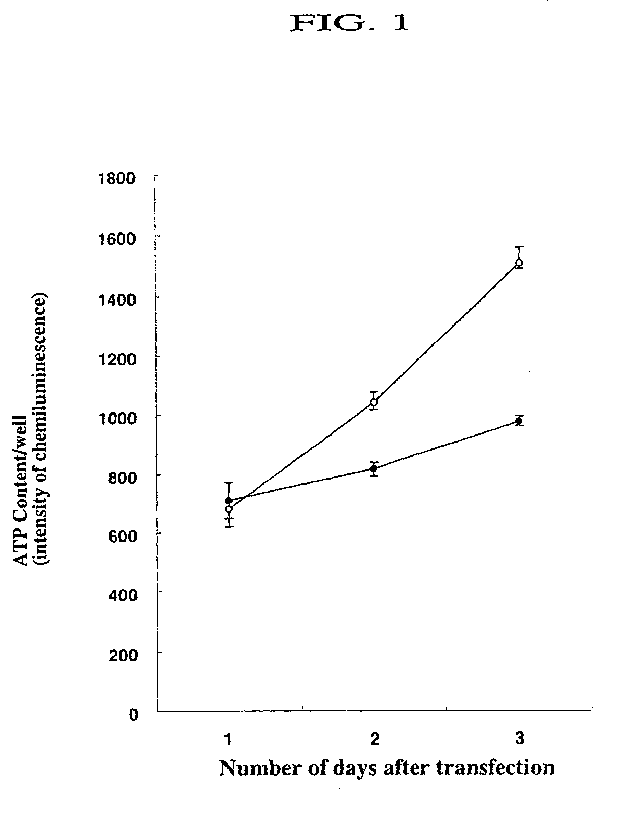

FIG. 1 shows the results of inhibited ATDC5 cell growth by forced expression of KIAA1077 gene. In the figure, the ordinate and the abscissa denote the chemiluminescence intensity reflecting the ATP content per well and the number of days after transfection, respectively. The open and closed circles designate control cells and KIAA1077-transfected cells, respectively, wherein n is 2. The bars designate the maximum and minimum values.

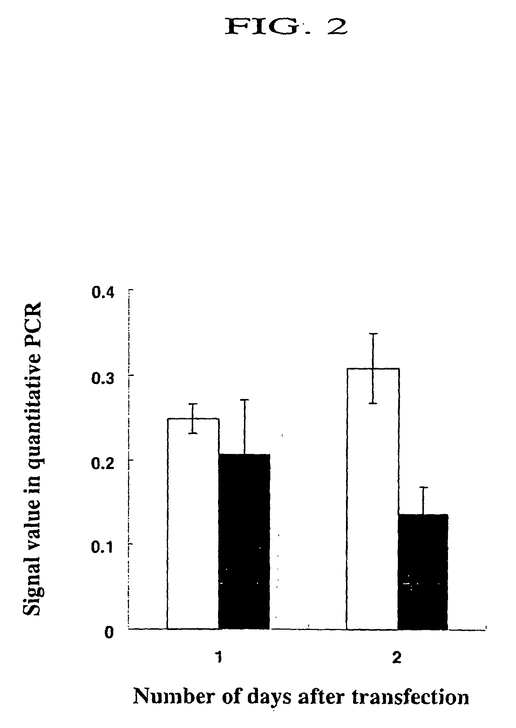

FIG. 2 shows the results of inhibited aggrecan expression of ATDC5 cells by forced expression of KIAA1077 gene. In the figure, the ordinate and the abscissa denote the chemiluminescence intensity reflecting the level of aggrecan gene-derived messenger RNA contained in 20 ng of total RNA and the number of days after transfection, respectively. The open and closed squares designate control cells and KIAA1077-transfected cells, respectively, wherein n is 3. The bars designate the standard deviation values.

### BEST MODE FOR CARRYING OUT THE INVENTION

The protein used in the present invention, which has the same or substantially the same amino acid sequence as the amino acid sequence represented by SEQ ID NO: 1 (hereinafter sometimes referred to as the protein of the present invention or the protein used in the present invention), may be any protein derived from any cells of human and warm-blooded animals (e.g., guinea pigs, rats, mice, fowl, rabbits, swine, sheep, bovine, monkeys, etc.) (e.g., hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; the proteins may also be synthetic proteins.

The amino acid sequence having substantially the same amino acid sequence as that represented by SEQ ID NO: 1 includes amino acid sequences having at least about 50% homology, preferably at least about 60% homology, more preferably at least about 70% homology, further more preferably at least about 80% homology, particularly preferably at least about 90% homology and most preferably at least about 95% homology, to the amino acid sequence shown by SEQ ID NO: 1; etc.

Preferred examples of the protein having substantially the same amino acid sequence as the amino acid sequence represented by SEQ ID NO: 1 include proteins having substantially the same amino acid sequence as the amino acid sequence represented by SEQ ID NO: 1 and having property substantially equivalent to that of the protein having the amino acid sequence represented by SEQ ID NO: 1, and the like.

The substantially equivalent properties include, for example, a sulfate ester hydrolyzing activity, and the like. The substantially equivalent is used to mean that the nature of these properties is equivalent in terms of quality (e.g., physiologically or pharmacologically). Therefore, the sulfate ester hydrolyzing activity is preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

The sulfate ester hydrolyzing activity can be determined by publicly known methods with modifications. For example, the activity can be assayed by using the method described in J. Inher. Metab. Dis., 16, 935-941, 1993 or by the method with modifications, using a fluorogenic substrate, 4-methyl umbelliferyl α-N-acetylglucosamine-6-sulfate. Alternatively, the sulfate ester hydrolysis activity can also be determined through quantification of sulfate ester hydrolysis product using an appropriate substrate, with utilization of the Morgan-Elson reaction.

For example, cells in which KIAA1077 cDNA-inserted plasmid is transfected and the KIAA1077 protein is abundantly expressed are incubated. After incubation the cells are recovered to prepare cell homogenate. The cell homogenate is mixed with the substrate and the mixture is kept warm, followed by the Morgan-Elson reaction. The sulfate ester hydrolysis activity is determined by assaying an increase of A₅₈₅ (absorption at 585 nm) in the reaction solution obtained.

As the substrate, there are employed, for example, sugar sulfates [e.g., chondroitin disaccharide Δdi-6S, chondroitin disaccharide Δdi-4S, sulfated glycosaminoglycans (e.g., chondroitin sulfate, keratan sulfate, dermatan sulfate, heparin, heparan sulfate, etc.) or their degradation products, etc.] and the like.

Examples of the protein used in the present invention include so-called muteins such as proteins containing (1) the amino acid sequence represented by SEQ ID NO: 1, of which at least 1 or 2 (preferably about 1 to about 30, more preferably about 1 to about 10 and most preferably several (1 to 5)) amino acids are deleted; (2) the amino acid sequence represented by SEQ ID NO: 1, to which at least 1 or 2 (preferably about 1 to about 30, more preferably about 1 to about 10 and most preferably several (1 to 5)) amino acids are added; (3) the amino acid sequence represented by SEQ ID NO: 1, in which at least 1 or 2 (preferably about 1 to about 30, more preferably about 1 to about 10 and most preferably several (1 to 5)) amino acids are inserted; (4) the amino acid sequence represented by SEQ ID NO: 1, in which at least 1 or 2 (preferably about 1 to about 30, more preferably about 1 to about 10 and most preferably several (1 to 5)) amino acids are substituted by other amino acids; or (5) mutains such as proteins with an amino acid sequence made of a combination of these amino acid sequences.

Where the amino acid sequences are inserted, deleted or substituted as described above, the position to be inserted, deleted or substituted is not particularly limited.

Throughout the specification, the proteins are represented in accordance with the conventional way of describing proteins, that is, the N-terminus (amino terminus) at the left hand and the C-terminus (carboxyl terminus) at the right hand. In the protein of the present invention including the protein containing the amino acid sequence shown by SEQ ID NO: 1, the C-terminus may be in any form of a carboxyl group (-COOH), a carboxylate (-COO-), an amide (-CONH₂) and an ester (-COOR).

Herein, examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group, e.g., benzyl, phenethyl, etc.; an α-naphthyl-C₁₋₂ alkyl group such as α-naphthylmethyl, etc.; pivaloyloxymethyl and the like.

Where the protein used in the present invention has a carboxyl group (or a carboxylate) at a position other than the C-terminus, the carboxyl group may be amidated or esterified and such an amide or ester is also included within the protein used in the present invention. Examples of the ester group in this case may be the C-terminal esters described above, etc.

Furthermore, examples of the protein used in the present invention include variants wherein the amino group at the N-terminal amino acid residues (e.g., methionine residue) is protected with a protecting group (e.g., a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆alkanoyl group, e.g., formyl group, acetyl group, etc.), or conjugated proteins such as glycoproteins having sugar chains; etc.

Specific examples of the protein used in the present invention are a protein (KIAA1077) containing the amino acid sequence represented by SEQ ID NO: 1, and the like.

The partial peptide of the protein used in the present invention may be any peptide as long as it is a partial peptide of the protein used in the present invention described above and preferably has the property equivalent to that of the protein used in the present invention described above.

Specifically, for the purpose of preparing the antibody of the present invention later described, there are peptides having 23-33 amino acid sequence in the amino acid sequence represented by SEQ ID NO: 1. For example, there are employed peptides having at least 20, preferably at least 50, more preferably at least 70, much more preferably at least 100, and most preferably at least 200 amino acids, in the amino acid sequence which constitutes the protein of the present invention.

The partial peptide used in the present invention may be peptides having the amino acid sequence, of which at least 1 or 2 (preferably about 1 to about 10 and more preferably several (1 to 5)) amino acids may be deleted; peptides, to which at least 1 or 2 (preferably about 1 to about 20, more preferably about 1 to about 10 and most preferably several (1 to 5)) amino acids may be added; in which at least 1 or 2 (preferably about 1 to about 20, more preferably about 1 to about 10 and most preferably several (1 to 5)) amino acids may be inserted; or, in which at least 1 or 2 (preferably about 1 to about 10, more preferably several and most preferably about 1 to about 5) amino acids may be substituted by other amino acids.

In the partial peptide used in the present invention, the C-terminus may be in any form of a carboxyl group (-COOH), a carboxylate (-COO-), an amide (-CONH₂) or an ester (-COOR).

Furthermore, the partial peptide used in the present invention includes variants having a carboxyl group (or a carboxylate) at a position other than the C-terminus, those wherein the amino group at the N-terminal amino acid residues (e.g., methionine residue) is protected with a protecting group; those wherein the N-terminal region is cleaved in vivo and the glutamyl group thus formed is pyroglutaminated; those wherein a substituent on the side chain of an amino acid in the molecule is protected with a suitable protecting group, or conjugated proteins such as so-called glycoproteins having sugar chains; etc., as in the protein used in the present invention described above.

The partial peptide used in the present invention may also be used as an antigen for producing antibodies.

As salts of the protein or partial peptide used in the present invention, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or partial peptide used in the present invention or salts thereof may be manufactured by a publicly known method used to purify a protein from human or warm-blooded animal cells or tissues described above. Alternatively, they may also be manufactured by culturing transformants containing DNAs encoding these proteins. Furthermore, they may also be manufactured by a modification of the methods for peptide synthesis, which will be described hereinafter.

Where these proteins are manufactured from human or mammalian tissues or cells, human or mammalian tissues or cells are homogenized, extracted with an acid or the like, and the extract is isolated and purified by a combination of chromatography techniques such as reverse phase chromatography, ion exchange chromatography, and the like.

To synthesize the protein or partial peptide used in the present invention or its salts, or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in the order of the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, and carbodiimides are particularly employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobomyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (linear, branched or cyclic alkyl esterification of, e.g., methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)], etc. As the amino acids in which the amino groups are activated in the starting material, for example, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein or partial peptide, for example, the α-carboxyl group of the carboxy terminal amino acid is first protected by amidation; the peptide (protein) chain is then extended from the amino group side to a desired length. Thereafter, a protein or partial peptide, in which only the protecting group of the N-terminal α-amino group of the peptide chain has been eliminated, and a protein or partial peptide, in which only the protecting group of the C-terminal carboxyl group has been eliminated are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide used in the present invention or salts thereof can be manufactured by publicly known methods for peptide synthesis, or by cleaving the protein used in the present invention with an appropriate peptidase. For the methods for peptide synthesis, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in (1) to (5) below.
(1) M. Bodanszky & M.A. Ondetti: Peptide Synthesis, Interscience Publishers, New York (1966)
(2) Schroeder & Luebke: The Peptide, Academic Press, New York (1965)
(3) Nobuo Izumiya, et al.: Peptide Gosei-no-Kiso to Jikken (Basics and experiments of peptide synthesis), published by Maruzen Co. (1975)
(4) Haruaki Yajima & Shunpei Sakakibara: Seikagaku Jikken Koza (Biochemical Experiment) 1, Tanpakushitsu no Kagaku (Chemistry of Proteins) IV, 205 (1977)
(5) Haruaki Yajima, ed.: Zoku Iyakuhin no Kaihatsu (A sequel to Development of Pharmaceutical), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the partial peptide can be converted into an appropriate salt by a publicly known method; when the partial peptide is obtained in a salt form, it can be converted into a free form or other different salt form by a publicly known method.

The polynucleotide encoding the protein used in the present invention may be any polynucleotide so long as it contains the base sequence encoding the protein used in the present invention described above. Preferably, the polynucleotide is DNA. Such a DNA may be any one of genomic DNA, genomic DNA library, cDNA derived from the cells/tissues described above, cDNA library derived from the cells/tissues described above and synthetic DNA.

The vector to be used for the library may be any of bacteriophage, plasmid, cosmid, phagemid, ane the like. The DNA may also be directly amplified by reverse transcriptase polymerase chain reaction (hereinafter abbreviated as RT-PCR) using the total RNA or mRNA fraction prepared from the cells/tissues described above.

The DNA encoding the protein used in the present invention may be, for example, any one of a DNA having the base sequence represented by SEQ ID NO: 2, or any DNA having a base sequence hybridizable to a DNA containing the base sequence represented by SEQ ID NO: 2 under high stringent conditions and encoding a protein which has the properties substantially equivalent to those of the protein containing the amino acid sequence represented by SEQ ID NO: 1.

Specific examples of the DNA that is hybridizable to the base sequence represented by SEQ ID NO: 2 under high stringent conditions include DNAs having at least about 50% homology, preferably at least about 60% homology, more preferably at least about 70% homology, further more preferably at least about 80% homology, particularly preferably at least about 90% homology and most preferably at least about 95% homology, to the base sequence represented by SEQ ID NO: 2; etc.

The hybridization can be carried out by publicly known methods or by a modification thereof, for example, according to the method described in Molecular Cloning, 2nd. (J. Sambrook et al., Cold Spring Harbor Lab. Press, 1989). A commercially available library can also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, as the DNA encoding the protein having the amino acid sequence represented by SEQ ID NO: 1, there may be employed a DNA containing the base sequence represented by SEQ ID NO: 2, etc.

The polynucleotide encoding the partial peptide used in the present invention may be any polynucleotide so long as it contains the base sequence encoding the partial peptide used in the present invention described above. Preferably, the polynucleotide is a DNA. Such a DNA may be any one of genomic DNA, genomic DNA library, cDNA derived from the cells/tissues described above, cDNA library derived from the cells/tissues described above and synthetic DNA.

As the DNA encoding the partial peptide used in the present invention, there are employed, for example, a DNA having a part of the base sequence represented by SEQ ID NO: 2, or a DNA containing a base sequence hybridizable to the base sequence represented by SEQ ID NO: 2 under high stringent conditions and containing a part of DNA encoding a protein having the activities substantially equivalent to those of the protein of the present invention, and so on.

The DNA hybridizable to the base sequence represented by SEQ ID NO: 2 has the same significance as described above.

Methods for the hybridization and high stringent conditions that can be used are the same as those described above.

For cloning of DNAs that completely encode the protein or partial peptide used in the present invention (hereinafter sometimes merely referred to as the protein of the present invention in the description of cloning of DNAs encoding the protein and partial peptide and their expression), the DNA can be either amplified by PCR using synthetic DNA primers containing a part of the base sequence of the protein of the present invention, or the DNA inserted into an appropriate vector can be screened by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Conversion of the base sequence of DNA can be effected by publicly known methods such as the ODA-LA PCR method, the Gapped duplex method, the Kunkel method, etc., or its modification, using a publicly known kit available as Mutan™-super Express Km (manufactured by Takara Shuzo Co., Ltd.) or Mutan™-K (manufactured by Takara Shuzo Co., Ltd.), etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, it is preferred to use CMV (cytomegalovirus) promoter, SRα promoter, etc. Where the host is bacteria of the genus Escherichia, preferred examples of the promoter include trp promoter, lac promoter, recA promoter, λP_{L} promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. when bacteria of the genus Escherichia is used as the host; α -amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977), etc.

As the insect, for example, a larva of Bombyx mori can be used [Maeda et al., Nature, 315, 592 (1985)].

Examples of animal cells include monkey cell COS-7, Vero, Chinese hamster cell CHO (hereinafter referred to as CHO cell), dhfr gene-deficient Chinese hamster cell CHO (hereinafter simply referred to as CHO (dhfr) cell), mouse L cell, mouse AtT-20, mouse myeloma cell, mouse ATDC5 cell, rat GH3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55 (1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors containing the DNAs encoding the protein can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the protein of the present invention can be produced in the transformant, on the cell membrane of the transformant, or outside of the transformant.

The protein of the present invention can be separated and purified from the culture described above by the following procedures.

When the protein of the present invention is extracted from the bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc. Thus, the crude extract of the protein can be obtained. The buffer used for the procedures may contain a protein modifier such as urea or guanidine hydrochloride, or a surfactant such as Triton X-100™, etc. When the protein is secreted in the culture broth, the supernatant can be separated, after completion of the cultivation, from the bacteria or cell to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be appropriately modified to partially remove the polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

The antibodies to the protein or partial peptide of the present invention, or its salts may be any of polyclonal and monoclonal antibodies, as long as they are capable of recognizing the protein or partial peptide of the present invention, or its salts.

The antibodies to the protein or partial peptide of the present invention, or its salts (hereinafter they are sometimes collectively referred to as the protein of the present invention in the description of the antibodies) can be produced by a publicly known method of producing an antibody or antiserum, using the protein of the present invention as an antigen.

### [Preparation of monoclonal antibody]

### (a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are monkeys, rabbits, dogs, guinea pigs, mice, rats, sheep, goats and fowl, with the use of mice and rats being preferred.

In the preparation of monoclonal antibody-producing cells, a warm-blooded animal, e.g., mice, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495 (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally in 5% CO₂. The antibody titer of the culture supernatant of a hybridoma can be determined as in the assay for the antibody titer in antisera described above.

### (b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

### [Preparation of polyclonal antibody]

The polyclonal antibody of the present invention can be manufactured by publicly known methods or modifications thereof. For example, a warm-blooded animal is immunized with an immunogen (protein antigen) per se, or a complex of immunogen and a carrier protein is formed and a warm-blooded animal is immunized with the complex in a manner similar to the method described above for the manufacture of monoclonal antibodies. The product containing the antibody to the protein of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiency produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The antisense nucleotide having a complementary or substantially complementary base sequence to the base sequence of DNA encoding the protein or partial peptide used in the present invention (hereinafter these DNAs are sometimes collectively referred to as the DNA of the present invention in the description of antisense nucleotide) can be any antisense nucleotide, so long as it possesses a complementary or substantially complementary base sequence to the base sequence of the DNA of the present invention and capable of suppressing the expression of said DNA, but antisense DNA is preferred.

The base sequence substantially complementary to the DNA of the present invention may include, for example, a base sequence having at least about 70% homology, preferably at least about 80% homology, more preferably at least about 90% homology and most preferably at least about 95% homology, to the entire base sequence or to the partial base sequence (i.e., complementary strand to the DNA of the present invention), and the like. Especially in the entire base sequence of the complementary strand to the DNA of the present invention, (a) in the case of antisense nucleotides directed to translation inhibition, suited are antisense nucleotides having at least about 70% homology, preferably at least about 80% homology, more preferably at least about 90% homology and most preferably at least about 95% homology, to the complementary strand of the base sequence which encodes the N-terminal region of the protein of the present invention (e.g., the base sequence around the initiation codon), and (b) in the case of antisense nucleotides directed to RNA degradation by RNaseH, suited are antisense nucleotides having at least about 70% homology, preferably at least about 80% homology, more preferably at least about 90% homology and most preferably at least about 95% homology, to the complementary strand of the entire base sequence of the DNA of present invention bearing intron.

Specific examples include an antisense nucleotide containing the entire or part of a base sequence complementary or substantially complementary to a base sequence of DNA containing the base sequence represented by SEQ ID NO: 2, preferably an antisense nucleotide containing the entire or part of a base sequence complementary to a base sequence of DNA containing the base sequence represented by SEQ ID NO: 2 (more preferably, an antisense nucleotide containing the entire or part of a base sequence complementary to a base sequence of DNA containing the base sequence represented by SEQ ID NO: 2), etc.

The antisense nucleotide is generally constituted by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. Alternatively, the sugar of each nucleotide (deoxyribose) may be replaced by a chemically modified sugar structure such as 2'-O-methylation, etc., and the base part (pyrimidine, purine) may undergo chemical modification. The antisense nucleotides may be any one, as far as they are hybridizable to a DNA containing the base sequence represented by SEQ ID NO: 2. These antisense nucleotides may be synthesized using a publicly known DNA synthesizer, etc.

Hereinafter, the protein of the present invention, its partial peptide, or salts thereof (hereinafter sometimes merely referred to as the protein of the present invention), the DNA encoding the protein of the present invention or its partial peptide (hereinafter sometimes merely referred to as the DNA of the present invention), the antibody to the protein of the present invention, its partial peptide, or salts thereof (hereinafter sometimes referred to as the antibody of the present invention) and the antisense nucleotide to the DNA of the present invention (hereinafter sometimes merely referred to as the antisense nucleotide of the present invention) are specifically described for their applications.

The protein of the present invention can be utilized as a disease marker since expression of the protein increases in osteoarthritis cartilage. That is, the protein is useful as a marker for early diagnosis in osteoarthritis cartilage, for judgment of severity in conditions, or for predicted development of the diseases. Thus, pharmaceuticals comprising the antisense nucleotide of a gene encoding the protein of the present invention, the compound or its salt that inhibits the activity of the protein of the present invention, or the antibody to the protein of the present invention or its salt can be used as prophylactic/therapeutic agents for bone and joint diseases such as chondrodysplasia, bone dysplasia, osteoporosis, osteoarthritis, chronic articular rheumatism, arthritis, synovitis, metabolic arthropathy or sports-related joint disorders (e.g., tennis elbow, etc.) or the like.

### (1) Screening of drug candidate compounds for disease

Since the protein of the present invention provides increased expression in osteoarthritis cartilage and exhibits an effect of inhibiting the proliferation of chondrocytes and chondroprogenitors, the compound or its salt regulating (preferably inhibiting) the activity of the protein of the present invention can be used as prophylactic/therapeutic agents for bone and joint diseases such as chondrodysplasia, bone dysplasia, osteoporosis, osteoarthritis, chronic articular rheumatism, arthritis, synovitis, metabolic arthropathy or sports-related joint disorders (e.g., tennis elbow, etc.) or the like.

Accordingly, the protein of the present invention is useful as a reagent for screening the compound or its salt that regulates (preferably inhibits) the activity of the protein of the present invention.

That is, the present invention provides a method of screening the compound or its salt that regulates (preferably inhibits) the activity (e.g., the sulfatase activity, etc.) of the protein of the present invention, which comprises using the protein of the present invention.

More specifically, there is used, for example, a method of screening the compound or its salt that regulates (promotes or inhibits, preferably inhibits) the activity of the protein of the present invention, which comprises comparing (i) the sulfatase activity of a cell capable of producing the protein of the present invention with (ii) the sulfatase activity of a mixture of a cell capable of producing the protein of the present invention and a test compound.

Specifically in the screening method described above, for example, in the cases of (i) and (ii), the cell is incubated and then recovered to prepare cell homogenate. The cell homogenate is mixed with a substrate and the mixture is kept warm, followed by the Morgan-Elson reaction. An increase of A₅₈₅ (absorption at 585 nm) in the reaction solution obtained is measured for comparison.

As the substrate, there are employed, for example, sugar sulfates [e.g., chondroitin disaccharide Δdi-6S, chondroitin disaccharide Δdi-4S, sulfated glycosaminoglycans (e.g., chondroitin sulfate, keratan sulfate, dermatan sulfate, heparin, heparan sulfate, etc.) or their degradation products, etc.] and the like.

As the cell capable of producing the protein of the present invention, there are employed, e.g., the aforesaid host (transformant) transformed with a vector having the DNA encoding the protein of the present invention. Preferably, animal cells such as CHO cells, etc. are used as the host. For the screening, the transformant, in which the protein of the present invention has been expressed on the cell membrane, e.g., by culturing through the procedure described above, is preferably employed. Incubation of the cell capable of producing the protein of the present invention is carried out in the same way as in the incubation of the transformant of the present invention described above.

Examples of the test compound include peptides, proteins, non-peptide compounds, synthetic compounds, fermentation products, cell extracts, plant extracts, animal tissue extracts, etc. These compounds may be novel compounds or publicly known compounds.

For example, when a test compound increases the sulfatase activity in the case of (ii) by at least about 20%, preferably at least about 30% and more preferably at least about 50% as compared to the case of (i), the compound can be selected as a compound promoting the activity of the protein of the present invention; and when a test compound decreases the sulfatase activity in the case of (ii) by at least about 20%, preferably at least about 30% and more preferably at least about 50%, the compound can be selected as a compound inhibiting the activity of the protein of the present invention.

The compound having the activity of promoting the protein of the present invention is useful as a safe and low toxic pharmaceutical for potentiating the activity of the protein of the present invention.

The compound having the activity of inhibiting the protein of the present invention is useful as a safe and low toxic pharmaceutical for suppressing the physiological activity of the protein of the present invention, for example, as prophylactic/therapeutic agents for bone and joint diseases.

The compound or its salt obtained! using the screening method or screening kit of the present invention is a compound selected from, for example, peptides, proteins, non-peptide compounds, synthetic compounds, fermentation products, cell extracts, plant extracts, animal tissue extracts, plasma, etc. The salts of the compound used are those given above as the salts of the peptide of the present invention.

In addition, the gene encoding the protein of the present invention also provides increased expression in osteoarthritis cartilage and exhibits an effect of inhibiting the proliferation of chondrocytes and chondroprogenitors. Thus, the compound or its salt regulating (preferably inhibiting) the expression of the gene encoding the protein of the present invention can also be used as prophylactic/therapeutic agents for bone and joint diseases such as chondrodysplasia, bone dysplasia, osteoporosis, osteoarthritis, chronic articular rheumatism, arthritis, synovitis, metabolic arthropathy or sports-related joint disorders (e.g., tennis elbow, etc.) or the like.

Accordingly, the DNA of the present invention is useful as a reagent for screening the compound or its salt that regulates (preferably inhibits) the expression of the gene encoding the protein of the present invention.

The screening method further includes a method of screening, which comprises comparing (iii) the case wherein a cell capable of producing the protein of the present invention is incubated, with (iv) the case wherein a cell capable of producing the protein used in the present invention is incubated in the presence of a test compound.

In the method described above, the expression level of the gene described above (specifically, the level of the protein of the present invention or the level of mRNA encoding said protein) is determined in the cases of (iii) and (iv), followed by comparing the cases.

The test compound and the cell capable of producing the protein of the present invention are the same as given above.

The level of the protein can be determined by publicly known methods, e.g., by measuring the said protein present in the cell extract, etc., using an antibody capable of recognizing the protein of the present invention, in accordance with methods like western blot analysis, ELISA, etc., or their modifications.

The level of mRNA can be determined by publicly known methods such as Northern hybridization using as a probe, e.g., a nucleic acid containing the entire or a part of base sequence represented by SEQ ID NO: 2, PCR using as a primer, e.g., a nucleic acid containing the entire or a part of base sequence represented by SEQ ID NO: 2, or modifications thereof.

For example, when a test compound increases the gene expression level in the case of (iv) by at least about 20%, preferably at least about 30% and more preferably at least about 50% as compared to the case of (iii) described above, the compound can be selected as a compound promoting the expression of the gene encoding the protein of the present invention; and when a test compound inhibits (decreases) the gene expression level by at least about 20%, preferably at least about 30% and more preferably at least about 50%, the compound can be selected as a compound suppressing (inhibiting) the expression of the gene encoding the protein of the present invention.

The screening kit of the present invention comprises the protein or its partial peptide used in the present invention, or a salt thereof or the cell capable of producing the protein or its partial peptide used in the present invention.

The compound or its salt obtained using the screening method or screening kit of the present invention is the test compound described above or the compound selected from, for example, peptides, proteins, non-peptide compounds, synthetic compounds, fermentation products, cell extracts, plant extracts, animal tissue extracts, plasma, etc., which is a compound or its salt regulating the activity of the protein of the present invention (e.g., the sulfatase activity, etc.).

The salts of these compounds used are those given above as the salts of the protein of the present invention.

The compound or its salts which regulate (preferably inhibit) the activity of the protein of the present invention or the compound or its salts which regulate (preferably inhibit) are useful, respectively, as prophylactic/therapeutic agents for bone and joint diseases such as chondrodysplasia, bone dysplasia, osteoporosis, osteoarthritis, chronic articular rheumatism, arthritis, synovitis, metabolic arthropathy or sports-related joint disorders (e.g., tennis elbow, etc.) or the like.

Where the compound or its salt obtained by using the screening method or screening kit of the present invention is used as the prophylactic/therapeutic agent described above, the compound or its salt can be prepared into pharmaceutical preparations in a conventional manner.

Examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations may be prepared by methods publicly known. For example, the injectable preparations may be prepared by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate dissolution aid such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a dissolution aid such as benzyl benzoate, benzyl alcohol, etc. The injection thus prepared is preferably filled in an appropriate ampoule. The suppository used for rectal administration may be prepared by blending the aforesaid antibody or its salt with conventional bases for suppositories.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid antibody contained is generally 5 to 500 mg per dosage unit form; especially in the form of injection, it is preferred that the aforesaid antibody is contained in 5 to 100 mg and in 10 to 250 mg for the other forms.

Each composition described above may further contain other active components unless formulation with the antibody described above causes any adverse interaction.

Since the pharmaceutical preparations thus obtained are safe and low toxic, they can be administered to human or warm-blooded animal (e.g., mouse, rat, rabbit, sheep, swine, bovine, horse, fowl, cat, dog, monkey, chimpanzee, etc.) orally or parenterally.

The dose of the compound or its salt may vary depending upon its action, target disease, subject to be administered, route of administration, etc. For example, when the compound or its salt that regulates (preferably inhibits) the activity of the protein of the present invention is orally administered for the purpose of treating, e.g., osteoarthritis, the compound or its salt is generally administered to an adult (as 60 kg body weight) in a daily dose of about 0.1 to about 100 mg, preferably about 1.0 to about 50 mg and more preferably about 1.0 to about 20 mg. In parenteral administration, a single dose of said compound or its salt may vary depending upon subject to be administered, target disease, etc. When the compound or its salt that regulates (preferably inhibits) the activity of the protein of the present invention is administered to an adult (as 60 kg body weight) in the form of an injectable preparation for the purpose of treating, e.g., osteoarthritis, it is advantageous to administer the compound or its salt intravenously in a daily dose of about 0.01 to about 30 mg, preferably about 0.1 to about 20 mg, and more preferably about 0.1 to about 10 mg. For other animal species, the corresponding dose as converted per 60 kg weight can be administered.

### (2) Quantification for the protein of the present invention, it partial peptide or salts thereof

The antibody to the protein of the present invention (hereinafter sometimes merely referred to as the antibody of the present invention) is capable of specifically recognizing the protein of the present invention, and thus can be used for quantification of the protein of the present invention in a test sample fluid, in particular, for quantification by sandwich immunoassay; etc.

That is, the present invention provides:
(i) a method of quantifying the protein of the present invention in a test sample fluid, which comprises competitively reacting the antibody of the present invention, a test sample fluid and a labeled form of the protein of the present invention, and measuring the ratio of the labeled form of the protein of the present invention bound to said antibody; and,
(ii) a method of quantifying the protein of the present invention in a test sample fluid, which comprises reacting a test sample fluid simultaneously or continuously with the antibody of the present invention immobilized on a carrier and a labeled form of another antibody of the present invention, and then measuring the activity of the labeling agent on the immobilized carrier.

In the quantification method (ii) described above, it is preferred that one antibody is capable of recognizing the N-terminal region of the protein of the present invention, while another antibody is capable of reacting with the C-terminal region of the protein of the present invention.

The monoclonal antibody to the protein of the present invention (hereinafter sometimes referred to as the monoclonal antibody of the present invention) can be used to quantify the protein of the present invention. In addition, the protein can be detected by means of a tissue staining as well. For these purposes, the antibody molecule per se may be used or F(ab')₂, Fab' or Fab fractions of the antibody molecule may also be used.

The method for quantification of the protein of the present invention using the antibody of the present invention is not particularly limited. Any quantification method can be used, so long as the amount of antibody, antigen or antibody-antigen complex corresponding to the amount of antigen (e.g., the amount of the protein) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use the sandwich method described hereinafter.

Examples of the labeling agent used in the assay method using the labeling substance are radioisotopes, enzymes, fluorescent substances, luminescent substances, and the like. As the radioisotopes, there are used, e.g., [¹²⁵I], [¹³¹I], [³H], [¹⁴C], etc. The enzymes described above are preferably enzymes, which are stable and have a high specific activity, and include, e.g., β-galactosidase. β-glucosidase, an alkaline phosphatase, a peroxidase, malate dehydrogenase, etc. As the fluorescent substances, there are used, e.g., fluorescamine, fluorescein isothiocyanate, etc. As the luminescent substances described above there are used, e.g., luminol, a luminol derivative, luciferin, lucigenin, etc. Furthermore, the biotin-avidin system may be used as well for binding of an antibody or antigen to a labeling agent.

For immobilization of the antigen or antibody, physical adsorption may be used. Chemical binding techniques conventionally used for insolubilization or immobilization of proteins, enzymes, etc. may also be used. For carriers, there are used, e.g., insoluble polysaccharides such as agarose, dextran, cellulose, etc.; synthetic resin such as polystyrene, polyacrylamide, silicon, etc., and glass or the like.

In the sandwich method, the immobilized monoclonal antibody of the present invention is reacted with a test fluid (primary reaction), then with a labeled form of another monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more antibodies may be used to increase the measurement sensitivity.

In the methods of assaying the protein of the present invention by the sandwich method, antibodies that bind to different sites of the protein of the present invention are preferably used as the monoclonal antibodies of the present invention used for the primary and secondary reactions. That is, in the antibodies used for the primary and secondary reactions are, for example, when the antibody used in the secondary reaction recognizes the C-terminal region of the protein of the present invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, the competitive method, the immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the protein of the present invention or its salts are constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As described above, the protein of the present invention can be quantified with high sensitivity, using the antibody of the present invention.

Furthermore, when an increased or decreased level, preferably an increased level of the protein of the present invention is detected by quantifying the level of the protein of the present invention using the antibody of the present invention, it can be diagnosed that one suffers from bone and joint diseases, for example, chondrodysplasia, bone dysplasia, osteoporosis, osteoarthritis, chronic articular rheumatism, arthritis, synovitis, metabolic arthropathy or sports-related joint disorders (e.g., tennis elbow, etc.), etc.; or it is highly likely to suffer from these disease in the future.

Moreover, the antibody of the present invention can be used to detect the protein of the present invention, which is present in a test sample such as a body fluid, a tissue, etc. The antibody can also be used to prepare an antibody column for purification of the protein of the present invention, detect the protein of the present invention in each fraction upon purification, analyze the behavior of the protein of the present invention in the cells under investigation; etc.

### (3) Gene diagnostic agent

By using the DNA of the present invention, e.g., as a probe, an abnormality (gene abnormality) of the DNA or mRNA encoding the protein of the present invention in human or warm-blooded animal (e.g., rat, mouse, guinea pig, rabbit, fowl, sheep, swine, bovine, horse, cat, dog, monkey, chimpanzee, etc.) can be detected. Therefore, the DNA of the present invention is useful as a gene diagnostic agent for detecting damages to the DNA or mRNA, its mutation, or decreased expression, increased expression, overexpression, etc. of the DNA or mRNA, and so on.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

For example, when overexpression or reduced expression is detected by Northern hybridization or DNA mutation is detected by PCR-SSCP, it can be diagnosed that it is highly likely to suffer from bone and joint diseases, for example, chondrodysplasia, bone dysplasia, osteoporosis, osteoarthritis, chronic articular rheumatism, arthritis, synovitis, metabolic arthropathy or sports-related joint disorders (e.g., tennis elbow, etc.), etc.

### (4) Pharmaceutical comprising an antisense nucleotide

The antisense nucleotide of the present invention that complementarily binds to the DNA of the present invention thereby to inhibit the expression of the DNA is low toxic and can suppress the activity/function (e.g., the sulfatase activity) of the DNA of the present invention in vivo. Thus, the antisense nucleotide can be used as a prophylactic/therapeutic agent for bone and joint diseases, for example, chondrodysplasia, bone dysplasia, osteoporosis, osteoarthritis, chronic articular rheumatism, arthritis, synovitis, metabolic arthropathy or sports-related joint disorders (e.g., tennis elbow, etc.), etc.

Where the antisense nucleotide is used as the prophylactic/therapeutic agent described above, it can be prepared into pharmaceutical preparations by publicly known methods, which are provided for administration.

For example, when the antisense nucleotide is used, the antisense nucleotide alone is administered directly, or the antisense nucleotide is inserted into an appropriate vector such as retrovirus vector, adenovirus vector, adenovirus-associated virus vector, etc., followed by treating in a conventional manner. The antisense nucleotide may then be administered orally or parenterally to human or mammal (e.g., rat, rabbit, sheep, swine, bovine, cat, dog, monkey, etc.) in a conventional manner. The antisense nucleotide may also be administered as it stands, or may be prepared in pharmaceutical preparations together with a physiologically acceptable carrier to assist its uptake, which are then administered by gene gun or through a catheter such as a catheter with a hydrogel. Alternatively, the antisense nucleotide may be prepared into an aerosol, which is topically administered into the trachea as an inhaler.

Further for purposes of improving pharmacokinetics, prolonging a half-life and improving intracellular uptake efficiency, the antisense nucleotide described above is prepared into pharmaceutical preparations (injectable preparations) alone or together with a carrier such as liposome, etc. and the preparations may be administered intravenously, subcutaneously, intraarticularly, etc.

The dose of the antisense nucleotide may vary depending on target disease, subject to be administered, route for administration, etc. When the antisense nucleotide of the present invention is administered for the treatment of, e.g., osteoarthritis, the antisense nucleotide is generally administered to an adult (body weight of 60 kg) in a daily dose of about 0.1 to about 100 mg.

In addition, the antisense nucleotide may also be used as an oligonucleotide probe for diagnosis to examine the presence of the DNA of the present invention in tissues or cells and states of its expression.

As the antisense nucleotide described above can, double-stranded RNA containing a part of RNA encoding the protein of the present invention, ribozyme containing a part of RNA encoding the protein of the present invention and the like can suppress the expression of the gene of the present invention and can suppress the in vivo function of the protein used in the present invention or the DNA used in the present invention. Thus, they can be used as a prophylactic/therapeutic agent for bone and joint diseases, for example, chondrodysplasia, bone dysplasia, osteoporosis, osteoarthritis, chronic articular rheumatism, arthritis, synovitis, metabolic arthropathy or sports-related joint disorders (e.g., tennis elbow, etc.), etc.

The double-stranded RNA can be designed based on a sequence of the polynucleotide of the present invention and manufactured by modifications of publicly known methods (e.g., Nature, 411, 494, 2001).

The ribozyme can be designed based on a sequence of the polynucleotide of the present invention and manufactured by modifications of publicly known methods (e.g., TRENDS in Molecular Medicine, 7, 221, 2001). For example, the ribozyme can be manufactured by ligating a publicly known ribozyme to a part of the RNA encoding the protein of the present invention. A part of the RNA encoding the protein of the present invention includes an adjacent portion to a cleavage site on the RNA of the present invention, which may be cleaved by a publicly known ribozyme (RNA fragment).

Where the double-stranded RNA or ribozyme described above can be used as the preventive/therapeutic agent described above, the ribozyme can be prepared into pharmaceutical preparations, which are provided for administration, as in the antisense polynucleotide.

### (5) Pharmaceutical comprising the antibody of the present invention

The antibody having the action of neutralizing the activity of the protein of the present invention can be used as a prophylactic/therapeutic agent for bone and joint diseases such as chondrodysplasia, bone dysplasia, osteoporosis, osteoarthritis, chronic articular rheumatism, arthritis, synovitis, metabolic arthropathy or sports-related joint disorders (e.g., tennis elbow, etc.), etc.

The prophylactic/therapeutic agent described above comprising the antibody of the present invention is low toxic and can be administered to human or mammal (e.g., rat, rabbit, sheep, swine, bovine, cat, dog, monkey, etc.) orally or parenterally (e.g., intraarticularly) in the form of liquid as it is or as a pharmaceutical composition in a suitable preparation. The dose may vary depending upon subject to be administered, target disease, conditions, route of administration, etc. When it is used for the treatment/prevention of, e.g., osteoarthritis, it is advantageous to administer the antibody of the present invention intraarticularly to an adult in a single dose of about 0.01 to about 20 mg/kg body weight, preferably about 0.1 to about 10 mg/kg body weight, more preferably about 0.1 to about 5 mg/kg body weight, in about 1 to about 10 times per month, preferably in about 1 to about 3 times per month. In other parenteral administration and oral administration, the antibody can be administered in a dose corresponding to the above dose. When the condition is especially severe, the dose may be increased accordingly to the condition.

The antibody of the present invention may be administered in itself or as an appropriate pharmaceutical composition. The pharmaceutical composition used for the administration described above contains a pharmacologically acceptable carrier with the aforesaid antibody or its salts, a diluent or excipient. Such a composition is provided in the form of pharmaceutical preparations suitable for oral or parenteral administration (e.g., intraarticular administration). Preferably, the composition is provided in the form of an inhaler.

Each composition described above may further contain other active components unless formulation with the antibody described above causes any adverse interaction.

### (6) Regarding "prophylactic/therapeutic agents for bone and joint diseases comprising the compound or its salt, having the action of regulating (preferably inhibiting) the sulfatase activity" and "prophylactic/therapeutic agents for bone and joint diseases comprising the compound or its salt, having the action of regulating (preferably inhibiting) the expression of a sulfatase"

The "compound or its salt, having the action of regulating (preferably inhibiting) the sulfatase activity" can be any compound, so long as it has the action of regulating (preferably inhibiting) the sulfatase activity. The compound is used as a prophylactic/therapeutic agent for bone and joint diseases such as chondrodysplasia, bone dysplasia, osteoporosis, osteoarthritis, chronic articular rheumatism, arthritis, synovitis, metabolic arthropathy or sports-related joint disorders (e.g., tennis elbow), etc.

The "compound or its salt, having the action of regulating (preferably inhibiting) the expression of a sulfatase" can be any compound, so long as it has the action of regulating (preferably inhibiting) the expression of a sulfatase. The compound is used as a prophylactic/therapeutic agent for bone and joint diseases such as chondrodysplasia, bone dysplasia, osteoporosis, osteoarthritis, chronic articular rheumatism, arthritis, synovitis, metabolic: arthropathy or sports-related joint disorders (e.g., tennis elbow), etc.

The prophylactic/therapeutic agent can be manufactured in the same way as described above.

### (7) DNA transgenic animal

The present invention provides a non-human mammal bearing DNA encoding the protein of the present invention, which is exogenous (hereinafter sometimes briefly referred to as the exogenous DNA of the present invention) or its variant DNA (sometimes briefly referred to as the exogenous variant DNA of the present invention).

That is, the present invention provides:
(1) A non-human mammal bearing the exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector containing the exogenous DNA of the present invention or its variant DNA and capable of expressing in a mammal; etc.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, sheep, goat, rabbits, dogs, cats, guinea pigs, hamsters, mice, rats, etc. Above all, preferred are rodents, especially mice (e.g., C57B1/6 strain, DBA2 strain, etc. for a pure line and for a cross line, B6C3F₁ strain, BDF₁ strain B6D2F₁ strain, BALB/c strain, ICR strain, etc.), rats (Wistar, SD, etc.) or the like, since they are relatively short in ontogeny and life cycle from a standpoint of creating model animals for disease.

"Mammals" in a recombinant vector that can be expressed in the mammals include the aforesaid non-human mammals and human.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses the abnormal protein of the present invention and exemplified by the DNA that expresses a protein for suppressing the function of the normal protein of the present invention.

The exogenous DNA of the present invention may be any one of those derived from a mammal of the same species as, or a different species from, the mammal as the target animal. In transfecting the DNA of the present invention, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the protein of the present invention, there are Escherichia colt-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression described above include (1) promoters for DNA derived from viruses (e.g., simian virus, cytomegalovirus, Moloney leukemia virus, JC virus, breast cancer virus, poliovirus, etc.), and (2) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β -hydroxylase, thyroid peroxidase (TPO), protein chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human protein elongation factor 1α (EF-1 α) promoters, human and fowl β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal protein obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the produced animal will retain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, after confirming that the exogenous DNA is stably retained by crossing.

By transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

A homozygous animal having the introduced DNA on both of homologous chromosomes can be acquired and male and female of the animal can be bred so that all the progeny retain the DNA in excess.

In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the protein of the present invention and the pathological mechanism of the disease associated with the protein of the present invention and to investigate how to treat these diseases.

Furthermore, since a mammal transfected with the exogenous normal DNA of the present invention has a condition of increasing the protein of the present invention liberated, the animal can also be used for a screening test of a prophylactic/therapeutic agent for bone and joint diseases such as chondrodysplasia, bone dysplasia, osteoporosis, osteoarthritis, chronic articular rheumatism, arthritis, synovitis, metabolic arthropathy or sports-related joint disorders (e.g., tennis elbow), etc.

On the other hand, a non-human mammal having the exogenous abnormal DNA of the present invention can be passaged under normal breeding conditions as the DNA-bearing animal after confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the animal after DNA transfection means that all of the offspring of the prepared animal have the abnormal DNA of the present invention in all of the germinal and somatic cells. Such an offspring that inherited the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the protein of the present invention by inhibiting the functions of endogenous normal DNA. Therefore, the animal can be utilized as a pathologic model animal for such a disease. For example, using the transgenic animal expressing the abnormal DNA of the present invention, it is possible to elucidate the mechanism of the function inactive type inadaptability to the protein of the present invention and the pathological mechanism of the disease associated with the protein of the present invention and to investigate how to treat the disease.

Specifically, the transgenic animal of the present invention expressing the abnormal DNA of the present invention at a high level is expected to serve as an experimental model to elucidate the mechanism of the functional inhibition (dominant negative effect) of a normal protein by the abnormal protein of the present invention in the function inactive type inadaptability of the protein of the present invention.

Moreover, a mammal bearing the abnormal exogenous DNA of the present invention has a condition of increasing the protein of the present invention liberated and is also available for a screening test of the protein of the present invention or a prophylactic/therapeutic agent for bone and joint diseases such as chondrodysplasia, bone dysplasia, osteoporosis, osteoarthritis, chronic articular rheumatism, arthritis, synovitis, metabolic arthropathy or sports-related joint disorders (e.g., tennis elbow), etc.

Other potential applications of two kinds of the DNA transgenic animals of the present invention described above further include:
(1) use as a cell source for tissue culture;
(2) elucidation of the relation to a peptide that is specifically expressed or activated by the protein of the present invention, by direct analysis of DNA or RNA in tissues of the DNA transgenic animal of the present invention or by analysis of the peptide tissues expressed by the DNA;
(3) research on the function of cells derived from tissues that are usually cultured only with difficulty, using cells in tissues bearing the DNA cultured by a standard tissue culture technique;
(4) screening a drug that enhances the functions of cells using the cells described in (3) above; and,
(5) isolation and purification of the variant protein of the present invention and preparation of an antibody thereto.

Furthermore, clinical conditions of a disease associated wit the protein of the present invention, including the function inactive type inadaptability to the protein of the present invention can be determined by using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.

Further by withdrawing each organ from the DNA transgenic animal of the present invention, mincing the organ and degrading with a proteinase such as trypsin, etc., it is possible to obtain a free form of the DNA-transfected cell, culture the cells or establish the cell line of cultured cells. Furthermore, the DNA transgenic animal of the present invention can serve to identify cells capable of producing the protein of the present invention, and to study in association with apoptosis, differentiation or propagation or on the mechanism of signal transduction in these properties to inspect any abnormality therein. Thus, the DNA transgenic animal can provide an effective research material for the protein of the present invention and for investigation of its function and effect.

To develop a drug for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

### (9) Knockout animal

The present invention provides a non-human mammal embryonic stem cell bearing the DNA of the present invention inactivated and a non-human mammal deficient in expressing the DNA of the present invention.

Thus, the present invention provides:
(1) a non-human mammal embryonic stem cell in which the DNA of the present invention is inactivated;
(2) the embryonic stem cell according to (1), wherein the DNA is inactivated by introducing a reporter gene (e.g., β-galactosidase gene derived from *Escherichia coli*);
(3) the embryonic stem cell according to (1), which is resistant to neomycin;
(4) the embryonic stem cell according to (1), wherein the non-human mammal is a rodent;
(5) the embryonic stem cell according to (4), wherein the rodent is mouse;
(6) a non-human mammal deficient in expressing the DNA of the present invention, wherein the DNA is inactivated;
(7) the non-human mammal according to (6), wherein the DNA is inactivated by inserting a reporter gene (e.g., β-galactosidase derived from *Escherichia coli*) therein and the reporter gene is capable of being expressed under control of a promoter for the DNA of the present invention;
(8) the non-human mammal according to (6), which is a rodent;
(9) the non-human mammal according to (8), wherein the rodent is mouse; and,
(10) a method of screening a compound that promotes or inhibits (preferably inhibits) the promoter activity to the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).

As the non-human mammal, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter simply referred to as a targeting vector). The thus-obtained ES cells are subjected to the southern hybridization analysis using a DNA sequence on or near the DNA of the present invention as a probe, or to PCR analysis using as primers a DNA sequence on the targeting vector and another DNA sequence near the DNA of the present invention which is not included in the targeting vector, thereby to select the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the BDF₁ mouse (F₁ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about 10⁶ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1% trypsin/1 mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for cytological study of the protein of the present invention in vitro.

The non-human mammal deficient in expression of the DNA of the present invention can be identified from a normal animal by measuring the mRNA level in the subject animal by a publicly known method, and indirectly comparing the degrees of expression.

As the non-human mammal, the same examples given above are employed.

With respect to the non-human mammal deficient in expression of the DNA of the present invention, the DNA of the present invention can be made knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is rendered knockout permit passage rearing under ordinary rearing conditions, after the individuals obtained by their crossing have proven to be knockout.

Furthermore, the genital system may be obtained and retained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal, in which the DNA of the present invention is inactivated, lacks various biological activities derived from the protein of the present invention, such an animal can be a disease model suspected of inactivated biological activities of the protein of the present invention and thus, offers an effective study to investigate the causes for and therapy for these diseases.

### (9a) Method of screening a compound having a therapeutic/preventive effect on diseases caused by deficiency, damages, etc. of the DNA of the present invention

The non-human mammal deficient in expression of the DNA of the present invention can be employed for screening of a compound having a therapeutic/preventive effect on diseases caused by deficiency, damages, etc. of the DNA of the present invention.

That is, the present invention provides a method of screening a compound having a therapeutic/preventive effect on diseases caused by deficiency, damages, etc. of the DNA of the present invention, which comprises administering a test compound to a non-human mammal deficient in expression of the DNA of the present invention and, observing and measuring a change occurred in the animal.

As the non-human mammal deficient in expression of the DNA of the present invention used for the screening method, the same examples as given hereinabove are employed.

Examples of the test compound include peptides, proteins, non-peptide compounds, synthetic compounds, fermentation products, cell extracts, plant extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an indicator to assess the therapeutic/preventive effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately selected depending on the administration route, nature of the test compound, etc.

For screening of the compound having a preventive/therapeutic effect on bone and joint diseases such as chondrodysplasia, bone dysplasia, osteoporosis, osteoarthritis, chronic articular rheumatism, arthritis, synovitis, metabolic arthropathy or sports-related joint disorders (e.g., tennis elbow), etc., a test compound is administered to the non-human mammal deficient in expression of the DNA of the present invention; the joint, cartilage, etc. of the animal are monitored with passage of time to observe conditions of the diseases described above.

In the screening method, when a test compound is administered to a test animal and the above-described disease condition of the test animal are improved by at least about 10%, preferably at least about 30% and more preferably at least about 50%, the test compound can be selected to be a compound having a therapeutic/preventive effect on the diseases described above.

The compound obtained using the above screening method is a compound selected from the test compounds described above and exhibits a preventive/therapeutic effect on diseases caused by deficiencies, damages, etc. of the protein of the present invention. Therefore, the compound can be employed as a safe and low toxic drug for the prevention/treatment of the diseases. Furthermore, compounds derived from the compound obtained by the screening described above may be used as well.

The compound obtained by the screening method above may form salts, and may be used in the form of salts with physiologically acceptable acids (e.g., inorganic acids, organic acids, etc.) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

Pharmaceuticals comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or mammal (e.g., rat, mouse, guinea pig, rabbit, sheep, swine, bovine, horse, cat, dog, monkey, etc.).

The dose of the compound or its salt may vary depending upon target disease, subject to be administered, route of administration, etc. For example, when the compound is orally administered to an adult (as 60 kg body weight), the compound is generally administered to the patient with osteoarthritiss in a daily dose of about 0.1 to about 100 mg, preferably about 1.0 to about 50 mg and, more preferably about 1.0 to about 20 mg. In parenteral administration, a single dose of said compound or its salt may vary depending upon target subject, target disease, etc. When the compound is administered to an adult patient (as 60 kg body weight) with osteoarthritis in the form of an injectable preparation, it is advantageous to administer the compound intravenously to the patient in a daily dose of about 0.01 to about 30 mg, preferably about 0.1 to about 20 mg, and more preferably about 0.1 to about 10 mg. For other animal species, the corresponding dose as converted per 60 kg weight can be administered.

### (9b) Method of screening a compound that promotes or inhibits the activity of a promoter to the DNA of the present invention

The present invention provides a method of screening a compound or its salts that promote or inhibit the activity of a promoter to the DNA of the present invention, which comprises administering a test compound to a non-human mammal deficient in expression of the DNA of the present invention and detecting the expression of the reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, which is selected from the aforesaid non-human mammals deficient in expression of the DNA of the present invention.

The same examples as described above apply to the test compound.

As the reporter gene, the same specific examples apply to this screening method. Preferably, there are used β-galactosidase (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from *Escherichia coli,* β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method described above are compounds that are selected from the test compounds described above and that promote or inhibit the promoter activity to the DNA of the present invention.

The compound obtained by the screening method above may form salts, and may be used in the form of salts with physiologically acceptable acids (e.g., inorganic acids, etc.) or bases (e.g., organic acids, etc.) or the like, especially in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salt that promotes or inhibits the promoter activity to the DNA of the present invention can regulate the expression of the protein of the present invention and can regulate the function of the protein. Therefore, the compound or its salts are useful as prophylactic/therapeutic agents for bone and joint diseases such as chondrodysplasia, bone dysplasia, osteoporosis, osteoarthritis, chronic articular rheumatism, arthritis, synovitis, metabolic arthropathy or sports-related joint disorders (e.g., tennis elbow), etc.

In addition, compounds derived from the compound obtained by the screening described above may be used as well.

The pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or mammal (e.g., rat, mouse, guinea pig, rabbit, sheep, swine, bovine, horse, cat, dog, monkey, etc.).

A dose of the compound or its salt may vary depending on target disease, subject to be administered, route for administration, etc. For example, when the compound inhibiting the promoter activity to the DNA of the present invention is orally administered to an adult (as 60 kg body weight), the compound is administered to the patient with osteoarthritis normally in a daily dose of about 0.1 to 100 mg, preferably about 1.0 to 50 mg and more preferably about 1.0 to 20 mg. In parenteral administration, a single dose of the compound varies depending on subject to be administered, target disease, etc. but when the compound of inhibiting the promoter activity to the DNA of the present invention is administered to an adult (as 60 kg body weight) in the form of injectable preparation, it is advantageous to administer the compound intravenously to the patient with osteoarthritis in a daily dose of about 0.01 to about 30 mg, preferably about 0.1 to about 20 mg and more preferably about 0.1 to about 10 mg. For other animal species, the corresponding dose as converted per 60 kg weight can be administered.

As stated above, the non-human mammal deficient in expression of the DNA of the present invention is extremely useful for screening the compound or its salt that promotes or inhibits the promoter activity to the DNA of the present invention and, can greatly contribute to elucidation of causes for various diseases suspected of deficiency in expression of the DNA of the present invention and for development of prophylactic/therapeutic agents for these diseases.

Also, a so-called transgenic animal (gene transferred animal) can be prepared by using a DNA containing the promoter region of the protein of the present invention, ligating genes encoding various proteins at the downstream and injecting the same into oocyte of an animal. It is thus possible to synthesize the protein therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the protein of the present invention itself.

In the specification and drawings, the codes of bases, amino acids, etc. are denoted in accordance with the IUPAC-IUB Commission on Biochemical Nomenclature or by the common codes in the art, examples of which are shown below. For amino acids that may have the optical isomer, L form is presented unless otherwise indicated.
- DNA :: deoxyribonucleic acid
- cDNA :: complementary deoxyribonucleic acid
- A: : adenine
- T: : thymine
- G: : guanine
- C: : cytosine
- RNA :: ribonucleic acid
- mRNA :: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP: : adenosine triphosphate
- EDTA :: ethylenediaminetetraacetic acid
- SDS: : sodium dodecyl sulfate
- Gly: : glycine
- Ala: : alanine
- Val: : valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu: : pyroglutamic acid
- Sec: : selenocysteine

Substituents, protecting groups and reagents frequently used in this specification are presented as the codes below.
- Me: : methyl group
- Et: : ethyl group
- Bu: : butyl group
- Ph: : phenyl group
- TC :: thiazolidine-4(R)-carboxamido group
- Tos: : p-toluenesulfonyl
- CHO :: formyl
- Bzl: : benzyl
- Cl₂-Bzl :: 2,6-dichlorobenzyl
- Bom :: benzyloxymethyl
- Z: : benzyloxycarbonyl
- Cl-Z: : 2-chlorobenzyloxycarbonyl
- Br-Z: : 2-bromobenzyl oxycarbonyl
- Boc: : t-butoxycarbonyl
- DNP :: dinitrophenol
- Trt: : trityl
- Bum :: t-butoxymethyl
- Fmoc :: N-9-fluorenyl methoxycarbonyl
- HOBt: : 1-hydroxybenztriazole
- HOOBt :: 3,4-dihydro-3-hydroxy-4-oxo-1,2,3-benzotriazine
- HONB: :1-hydroxy-5-norbornene-2,3-dicarboxyimide
- DCC :: N,N'-dicyclohexylcarbodiimide

The sequence identification numbers in the sequence listing of the specification indicates the following sequence, respectively.

### [SEQ ID NO: 1]

This shows the amino acid sequence of KIAA1077 protein.

### [SEQ ID NO: 2]

This shows the base sequence of DNA encoding KIAA1077 protein having the amino acid sequence represented by SEQ ID NO: 1.

### [SEQ ID NO: 3]

This shows the base sequence of primer used in EXAMPLE 2.

### [SEQ ID NO: 4]

This shows the base sequence of primer used in EXAMPLE 2.

### [SEQ ID NO: 5]

This shows the base sequence of primer used in EXAMPLE 2.

### [SEQ ID NO: 6]

This shows the base sequence of primer used in EXAMPLE 2.

Hereinafter the present invention will be described more specifically with reference to EXAMPLES but is not deemed to be limited thereto.

### EXAMPLE 1

To clarify a group of genes with enhanced expression in the osteoarthritis cartilage (OA cartilage), gene expression analysis was performed on oligonucleotide microarrays (Human Genome U95A; Affymetrix Corp.), using as materials the total RNAs extracted from the OA cartilages (knee joint: 1 case, hip joint: 5 cases), normal joint cartilages (knee joint: 1 case, hip joint: 1 case) and other 21 normal tissues (1 case each) (TABLE 1).

Experiment was carried. out in accordance with the procedures in the expression analysis technical manual by Affymetrix Corp. The gene expression profiles were compared between the OA cartilages and normal joint cartilages. The results indicate that the expression of KIAA1077 gene was enhanced in the OA cartilages and in all the tissues analyzed, the KIAA1077 gene showed the maximum expression level in the OA cartilages (TABLE 2).

**[TABLE 1]**

| Tissue from which RNA was extracted | Distribution source |
|---|---|
| Cartilage in gonarthrosis | Direct Clinical Access, Inc. |
| Cartilage in osteoarthritis of the hip joint | Direct Clinical Access, Inc. |
| Cartilage in normal knee joint | Direct Clinical Access, Inc. |
| Normal hip joint | Direct Clinical Access, Inc. |
| Costal cartilage | BioChain Institute, Inc. |
| Fat | BioChain Institute, Inc. |
| Skeletal muscle | Clontech Laboratories, Inc. |
| Heart | Clontech Laboratories, Inc. |
| Kidney | Clontech Laboratories, Inc. |
| Adrenal | Clontech Laboratories, Inc. |
| Liver | Clontech Laboratories, Inc. |
| Pancreas | Clontech Laboratories, Inc. |
| Spleen | Clontech Laboratories, Inc. |
| Trachea | Clontech Laboratories, Inc. |
| Lung | Clontech Laboratories, Inc. |
| Whole brain | Clontech Laboratories, Inc. |
| Cerebellum | Clontech Laboratories, Inc. |
| Thyroid | Clontech Laboratories, Inc. |
| Thymus | Clontech Laboratories, Inc. |
| Mammary gland | Clontech Laboratories, Inc. |
| Stomach | Clontech Laboratories, Inc. |
| Rectum | BioChain Institute, Inc. |
| Colon | BioChain Institute, Inc. |
| Uterus | Clontech Laboratories, Inc. |
| Uterine cervix | BioChain Institute. Inc. |

**[TABLE 2]**

| Tissue | Gene expression level^{a} |
|---|---|
| Cartilage in gonarthrosis | 3.1^{b} |
| Cartilage in normal knee joint | ND |
| Normal hip joint | ND |
| Costal cartilage | 0.3 |
| Fat | 0.7 |
| Skeletal muscle | ND |
| Heart | ND |
| Spleen | ND |
| Thymus | ND |
| Whole brain | ND |
| Cerebellum | ND |
| Trachea | ND |
| Lung | ND |
| Adrenal | ND |
| Thyroid | 0.5 |
| Pancreas | ND |
| Stomach | ND |
| Liver | ND |
| Rectum | 0.5 |
| Colon | 0.5 |
| Kidney | ND |
| Mammary gland | ND |
| Uterus | 0.7 |
| Uterine cervix | ND |
| ND: not detected | |

| | |
|---|---|
| a: The medial value for the expression level of all genes, which expression was detected by the oligonucleotide microarrays was taken as 1 to standardize the gene expression level. | |
| b: Shown by a mean value (n=6). | |

### EXAMPLE 2

(1) Based on the base sequence of KIAA1077, GenBank Accession No. XM_053496, two synthetic primers (SEQ ID NO: 3 and SEQ ID NO: 4) were designed. Using the two synthetic primers, PCR was carried out using as template cDNAs derived from various human tissues (Clontech Laboratories, Inc.). As a thermoresistant DNA polymerase PfuTurbo purchased from Stratagene Corp. was used. The composition of reaction solution was in accordance with the manual of PfuTurbo. First, heating was carried out at 95°C for 2 minutes. Then, the amplification process of 98°C for 10 seconds - 65°C for 30 seconds - 72°C for 3 minutes and 30 seconds was performed in 35 cycles, and finally incubation at 72°C for 10 minutes was performed. The putative PCR product of about 3 kbp was noted in the reaction solution obtained from the brain cDNA. They were mixed and the PCR product amplified was purified on Strataprep PCR purification kit (Stratagene Corp.). The obtained DNA fragment of about 3 kb was cloned to DH5α using pCR-Script cloning kit (Stratagene Corp.). From the resulting Escherichia coli, plasmid DNA was isolated and the base sequence of DNA fragment of about 3 kb inserted into the cloning site was determined by the fluorescent Dye-Terminator method. In the base sequence obtained, an open reading frame (SEQ ID NO: 2) containing the base sequence (GenBank Accession No. XM_ 053496) of KIAA1077 gene was found between the two primer sequences used for PCR. The amino acid sequence encoded by the base sequence represented by SEQ ID NO: 2 is the sequence represented by SEQ ID NO: 1.

Using restriction enzymes Bam HI and Not I, cDNA of KIAA1077 was excised out of the obtained plasmid pCR-Script-K1077 and inserted into the Barn HI and Not I cleavage site of expression plasmid pcDNA5/TO (Invitrogen Corp.) in the mammalian (cell) system to acquire expression vector pcDNA5/TO-K1077.

(2) Mouse chondroprogenitor cell line ATDC5 cells (J. Bone Miner. Res., 12, 1174-1188, 1997) were plated on a 12-well culture plate at a density of 5 x 10⁴ cells/well. On the following day, pcDNA5/TO (Invitrogen Corp.) or the expression vector pcDNA5/TO-K1077 acquired in (1) above was transfected to ATDC5 cells in the amount of 0.7 µg DNA/well, respectively. GeneJammer transfection reagent (Stratagene Corp.) was used as a transfection reagent. Eight hours after, the cells were washed with phosphate-buffered saline and incubation was continued in DMEM/F-12 medium supplemented with 10% calf serum and 100 µg/ml of kanamycin. On 1, 2 and 3 days after the transfection, the cells were lysed in Cell Titer-Glo buffer (Promega Corp.) and stored at -80°C. These samples were thawed. After mixing with a Cell Titer-Glo substrate solution (Promega Corp.), chemiluminescence was measured to determine the ATP content in each sample. The ATP content was used as an indicator of the cell number (J. Immunol. Methods., 160, 81-88, 1993). The cell number of cells transfected with pcDNA5/TO (control cells) was compared with the cell number of cells transfected with pcDNA5/TO-K1077 (KIAA1077-transfected cells). The results indicate that the cells were equal in the ATP content/well one day after the transfection but 2 and 3 days after, the ATP content of the KIAA1077 expression cells per well was lower than that of control cells (FIG. 1).

From the results it was shown that growth of ATDC5 cells was suppressed by the KIAA1077 gene transfection.

(3) Mouse chondroprogenitor cell tine ATDC5 cells (J. Bone Miner. Res., 12, 1174-1188, 1997) were plated on a 12-well culture plate at a density of 5 x 10⁴ cells/well. On the following day, pcDNA5/TO (Invitrogen Corp.) or the expression vector pcDNA5/TO-K1077 acquired in (1) above was transfected to ATDC5 cells in the amount of 0.7 µg DNA/well, respectively. GeneJammer transfection reagent (Stratagene Corp.) was used as a transfection reagent. Eight hours after, the cells were washed with phosphate-buffered saline and incubation was continued in DMEM/F-12 medium supplemented with 10% calf serum and 100 µg/ml of kanamycin. On 1 and 2 days after the transfection, the cells were lysed in RLT buffer (Qiagen, Inc.), then treated with QIAshredder (Qiagen, Inc.) and stored at -80°C. After the sample was thawed, total RNA derived from the cells was prepared using RNeasy mini kit (Qiagen, Inc.). In this case, Dnase treatment on a column was performed in accordance with the option protocol to remove any trace of genomic DNA contamination. Using the total RNA obtained as a template, quantitative RT-PCR was carried out to make comparison on the expression level of aggrecan gene. Based on the base sequence gi/6671522/ ref/NM_007424 registered in GenBank, 2 primers (SEQ ID NO: 5 and SEQ ID NO: 6) for mouse aggrecan mRNA were designed and provided for use. Using Quanti-Tect SYBR green RT-PCR kit (Qiagen, Inc.) as a reagent, RT-PCR, signal detection and data analysis were performed on 7700 sequence detection system (Applied Biosystems, Inc.). First, incubation at 50°C for 30 minutes was carried out, and followed by heating at 95°C for 15 minutes. Subsequently, the amplification process of 94°C for 15 seconds and 60°C for 60 seconds was performed in 40 cycles. On each of the samples derived from pcDNA5/TO-transfected cells (control cells) and pcDNA5/TO-K1077-transfected cells (KIAA1077-introduced cells), 20 ng of total RNA was analyzed to compare control cells with the KIAA1077-introduced cells. The results indicate a tendency to lower the expression level of aggrecan gene in the KIAA1077-introduced cells 1 day after transfection and 2 days after the expression level was lowered to 50% or less as compared to control cells (FIG. 2).

From the results it was shown that the expression of endogenous aggrecan gene in ATDC5 cells was suppressed by KIAA1077 gene transfection.

### INDUSTRIAL APPLICABILITY

The protein used in the present invention is increasingly expressed in osteoarthritis cartilage, has an effect of inhibiting the proliferation of chondrocytes and chondroprogenitors. Accordingly, the protein is useful as a diagnostic marker for, e.g., bone and joint diseases. The compound or its salt, which regulates (preferably inhibits) the activity of the protein, the compound or its salt, which regulates (preferably inhibits) the expression of a gene for the protein, a neutralizing antibody to the protein and the antisense nucleotide of the present invention can be safely used as prophylactic/therapeutic agents for bone and joint diseases, for example, chondrodysplasia, bone dysplasia, osteoporosis, osteoarthritis, chronic articular rheumatism, arthritis, synovitis, metabolic arthropathy or sports-related joint disorders (e.g., tennis elbow), etc.

## Claims

1. A prophylactic/therapeutic agent for bone and joint diseases, comprising a compound or its salt that inhibits the activity of a protein having an amino acid sequence which is the same or substantially the same as the amino acid sequence represented by SEQ ID NO: 1, its partial peptide, or a salt thereof.

2. A prophylactic/therapeutic agent for bone and joint diseases, comprising a compound or its salt that inhibits the expression of a gene for a protein having an amino acid sequence which is the same or substantially the same as the amino acid sequence represented by SEQ ID NO: 1, its partial peptide, or a salt thereof.

3. A prophylactic/therapeutic agent for bone and joint diseases, comprising an antisense nucleotide having a base sequence which is complementary or substantially complementary to the base sequence of a polynucleotide encoding a protein having an amino acid sequence which is the same or substantially the same as the amino acid sequence represented by SEQ ID NO: 1, or its partial peptide, or a part of the base sequence.

4. A prophylactic/therapeutic agent for bone and joint diseases, comprising an antibody to a protein having an amino acid sequence which is the same or substantially the same as the amino acid sequence represented by SEQ ID NO: 1, its partial peptide, or a salt thereof.

5. The prophylactic/therapeutic agent according to claim 1, 2, 3 or 4, wherein the bone and joint diseases are chondrodysplasia, bone dysplasia, osteoporosis, osteoarthritis, chronic articular rheumatism, arthritis, synovitis, metabolic arthropathy or sports-related joint disorders.

6. A diagnostic agent for bone and joint diseases, comprising an antibody to a protein having an amino acid sequence which is the same or substantially the same as the amino acid sequence represented by SEQ ID NO: 1, its partial peptide, or a salt thereof.

7. A diagnostic agent for bone and joint diseases, comprising a polynucleotide encoding a protein having an amino acid sequence which is the same or substantially the same as the amino acid sequence represented by SEQ ID NO: 1, or its partial peptide.

8. The diagnostic agent according to claim 6 or 7, wherein the bone and joint diseases are chondrodysplasia, bone dysplasia, osteoporosis, osteoarthritis, chronic articular rheumatism, arthritis, synovitis, metabolic arthropathy or sports-related joint disorders.

9. A prophylactic/therapeutic agent for bone and joint diseases, comprising a compound or its salt having an action of inhibiting a sulfatase activity.

10. A prophylactic/therapeutic agent for bone and joint diseases, comprising a compound or its salt having an action of inhibiting the expression of a sulfatase.

11. A method of screening a prophylactic/therapeutic agent for bone and joint diseases, which comprises using a protein having an amino acid sequence which is the same or substantially the same as the amino acid sequence represented by SEQ ID NO: 1, its partial peptide, or a salt thereof.

12. A kit for screening a prophylactic/therapeutic agent for bone and joint diseases, comprising a protein having an amino acid sequence which is the same or substantially the same as the amino acid sequence represented by SEQ ID NO: 1, its partial peptide, or a salt thereof.

13. A method of screening a prophylactic/therapeutic agent for bone and joint diseases, which comprises using a polynucleotide encoding a protein having an amino acid sequence which is the same or substantially the same as the amino acid sequence represented by SEQ ID NO: 1, or its partial peptide.

14. A kit for screening a prophylactic/therapeutic agent for bone and joint diseases, comprising a polynucleotide encoding a protein having an amino acid sequence which is the same or substantially the same as the amino acid sequence represented by SEQ ID NO: 1, or its partial peptide.

15. The prophylactic/therapeutic agent for bone and joint diseases, which is obtainable using the screening method according to claim 11 or 13, or the screening kit according to claim 12 or 14.

16. A method of preventing/treating bone and joint diseases, which comprises administering to a mammal an effective dose of a compound or its salt that inhibits the activity of a protein having an amino acid sequence which is the same or substantially the same as the amino acid sequence represented by SEQ ID NO: 1, its partial peptide, or a salt thereof, or an effective dose of a compound or its salt that inhibits the expression of a gene for the protein.

17. A method of preventing/treating bone and joint diseases, which comprises inhibiting the activity of a protein having an amino acid sequence which is the same or substantially the same as the amino acid sequence represented by SEQ ID NO: 1, its partial peptide, or a salt thereof, or inhibiting the expression of a gene for the protein.

18. Use of a compound or its salt that inhibits the activity of a protein having an amino acid sequence which is the same or substantially the same as the amino acid sequence represented by SEQ ID NO: 1, its partial peptide, or a salt thereof or a compound or its salt that inhibits the expression of a gene for the protein, to manufacture a prophylactic/therapeutic agent for bone and joint diseases.


SEQUENCE LISTING

<110> Takeda Chemical Industries, Ltd.

<120> Prophylactic/therapeutic agents for bone or joint diseases

<130> P04-166US,EP

<150> JP2002-178715
<151> 2002-6-19

<160> 6

<210> 1
<211> 871
<212> PRT
<213> Homo sapiens

<400> 1
Met Lys Tyr Ser Cys Cys Ala Leu Val Leu Ala Val Leu Gly Thr Glu
                5                   10                  15
Leu Leu Gly Ser Leu Cys Ser Thr Val Arg Ser Pro Arg Phe Arg Gly
            20                  25                  30
Arg Ile Gln Gln Glu Arg Lys Asn Ile Arg Pro Asn Ile Ile Leu Val
        35                  40                  45
Leu Thr Asp Asp Gln Asp Val Glu Leu Gly Ser Leu Gln Val Met Asn
    50                  55                  60
Lys Thr Arg Lys Ile Met Glu His Gly Gly Ala Thr Phe Ile Asn Ala
65                  70                  75                  80
Phe Val Thr Thr Pro Met Cys Cys Pro Ser Arg Ser Ser Met Leu Thr

85                    90                    95
Gly Lys Tyr Val His Asn His Asn Val Tyr Thr Asn Asn Glu Asn Cys
            100                   105                   110
Ser Ser Pro Ser Trp Gln Ala Met His Glu Pro Arg Thr Phe Ala Val
        115                   120                   125
Tyr Leu Asn Asn Thr Gly Tyr Arg Thr Ala Phe Phe Gly Lys Tyr Leu
    130                   135                   140
Asn Glu Tyr Asn Gly Ser Tyr Ile Pro Pro Gly Trp Arg Glu Trp Leu
145                   150                   155                   160
Gly Leu Ile Lys Asn Ser Arg Phe Tyr Asn Tyr Thr Val Cys Arg Asn
                165                   170                   175
Gly Ile Lys Glu Lys His Gly Phe Asp Tyr Ala Lys Asp Tyr Phe Thr
            180                   185                   190
Asp Leu Ile Thr Asn Glu Ser Ile Asn Tyr Phe Lys Met Ser Lys Arg
        195                   200                   205
Met Tyr Pro His Arg Pro Val Met Met Val Ile Ser His Ala Ala Pro
    210                   215                   220
His Gly Pro Glu Asp Ser Ala Pro Gln Phe Ser Lys Leu Tyr Pro Asn
225                   230                   235                   240
Ala Ser Gln His Ile Thr Pro Ser Tyr Asn Tyr Ala Pro Asn Met Asp
                245                   250                   255
Lys His Trp Ile Met Gln Tyr Thr Gly Pro Met Leu Pro Ile His Met
            260                   265                   270
Glu Phe Thr Asn Ile Leu Gln Arg Lys Arg Leu Gln Thr Leu Met Ser
        275                   280                   285
Val Asp Asp Ser Val Glu Arg Leu Tyr Asn Met Leu Val Glu Thr Gly
    290                   295                   300
Glu Leu Glu Asn Thr Tyr Ile Ile Tyr Thr Ala Asp His Gly Tyr His
305                   310                   315                   320
Ile Gly Gln Phe Gly Leu Val Lys Gly Lys Ser Met Pro Tyr Asp Phe

                325                 330                 335
Asp Ile Arg Val Pro Phe Phe Ile Arg Gly Pro Ser Val Glu Pro Gly
            340                 345                 350
Ser Ile Val Pro Gln Ile Val Leu Asn Ile Asp Leu Ala Pro Thr Ile
        355                 360                 365
Leu Asp Ile Ala Gly Leu Asp Thr Pro Pro Asp Val Asp Gly Lys Ser
    370                 375                 380
Val Leu Lys Leu Leu Asp Pro Glu Lys Pro Gly Asn Arg Phe Arg Thr
385                 390                 395                 400
Asn Lys Lys Ala Lys Ile Trp Arg Asp Thr Phe Leu Val Glu Arg Gly
                405                 410                 415
Lys Phe Leu Arg Lys Lys Glu Glu Ser Ser Lys Asn Ile Gln Gln Ser
            420                 425                 430
Asn His Leu Pro Lys Tyr Glu Arg Val Lys Glu Leu Cys Gln Gln Ala
        435                 440                 445
Arg Tyr Gln Thr Ala Cys Glu Gln Pro Gly Gln Lys Trp Gln Cys Ile
    450                 455                 460
Glu Asp Thr Ser Gly Lys Leu Arg Ile His Lys Cys Lys Gly Pro Ser
465                 470                 475                 480
Asp Leu Leu Thr Val Arg Gln Ser Thr Arg Asn Leu Tyr Ala Arg Gly
                485                 490                 495
Phe His Asp Lys Asp Lys Glu Cys Ser Cys Arg Glu Ser Gly Tyr Arg
            500                 505                 510
Ala Ser Arg Ser Gln Arg Lys Ser Gln Arg Gln Phe Leu Arg Asn Gln
        515                 520                 525
Gly Thr Pro Lys Tyr Lys Pro Arg Phe Val His Thr Arg Gln Thr Arg
    530                 535                 540
Ser Leu Ser Val Glu Phe Glu Gly Glu Ile Tyr Asp Ile Asn Leu Glu
545                 550                 555                 560
Glu Glu Glu Glu Leu Gln Val Leu Gln Pro Arg Asn Ile Ala Lys Arg

                565                 570                 575
His Asp Glu Gly His Lys Gly Pro Arg Asp Leu Gln Ala Ser Ser Gly
            580                 585                 590
Gly Asn Arg Gly Arg Met Leu Ala Asp Ser Ser Asn Ala Val Gly Pro
        595                 600                 605
Pro Thr Thr Val Arg Val Thr His Lys Cys Phe Ile Leu Pro Asn Asp
    610                 615                 620
Ser Ile His Cys Glu Arg Glu Leu Tyr Gln Ser Ala Arg Ala Trp Lys
625                 630                 635                 640
Asp His Lys Ala Tyr Ile Asp Lys Glu Ile Glu Ala Leu Gln Asp Lys
                645                 650                 655
Ile Lys Asn Leu Arg Glu Val Arg Gly His Leu Lys Arg Arg Lys Pro
            660                 665                 670
Glu Glu Cys Ser Cys Ser Lys Gln Ser Tyr Tyr Asn Lys Glu Lys Gly
        675                 680                 685
Val Lys Lys Gln Glu Lys Leu Lys Ser His Leu His Pro Phe Lys Glu
    690                 695                 700
Ala Ala Gln Glu Val Asp Ser Lys Leu Gln Leu Phe Lys Glu Asn Asn
705                 710                 715                 720
Arg Arg Arg Lys Lys Glu Arg Lys Glu Lys Arg Arg Gln Arg Lys Gly
                725                 730                 735
Glu Glu Cys Ser Leu Pro Gly Leu Thr Cys Phe Thr His Asp Asn Asn
            740                 745                 750
His Trp Gln Thr Ala Pro Phe Trp Asn Leu Gly Ser Phe Cys Ala Cys
        755                 760                 765
Thr Ser Ser Asn Asn Asn Thr Tyr Trp Cys Leu Arg Thr Val Asn Glu
    770                 775                 780
Thr His Asn Phe Leu Phe Cys Glu Phe Ala Thr Gly Phe Leu Glu Tyr
785                 790                 795                 800
Phe Asp Met Asn Thr Asp Pro Tyr Gln Leu Thr Asn Thr Val His Thr

                805                 810                 815
Val Glu Arg Gly Ile Leu Asn Gln Leu His Val Gln Leu Met Glu Leu
            820                 825                 830
Arg Ser Cys Gln Gly Tyr Lys Gln Cys Asn Pro Arg Pro Lys Asn Leu
        835                 840                 845
Asp Val Gly Asn Lys Asp Gly Gly Ser Tyr Asp Leu His Arg Gly Gln
    850                 855                 860
Leu Trp Asp Gly Trp Glu Gly
865                 870

<210> 2
<211> 2613
<212> DNA
<213> Homo sapiens

<400> 2
atgaagtatt cttgctgtgc tctggttttg gctgtcctgg gcacagaatt gctgggaagc 60
ctctgttcga ctgtcagatc cccgaggttc agaggacgga tacagcagga acgaaaaaac 120
atccgaccca acattattct tgtgcttacc gatgatcaag atgtggagct ggggtccctg 180
caagtcatga acaaaacgag aaagattatg gaacatgggg gggccacctt catcaatgcc 240
tttgtgacta cacccatgtg ctgcccgtca cggtcctcca tgctcaccgg gaagtatgtg 300
cacaatcaca atgtctacac caacaacgag aactgctctt ccccctcgtg gcaggccatg 360
catgagcctc ggacttttgc tgtatatctt aacaacactg gctacagaac agcctttttt 420
ggaaaatacc tcaatgaata taatggcagc tacatccccc ctgggtggcg agaatggctt 480
ggattaatca agaattctcg cttctataat tacactgttt gtcgcaatgg catcaaagaa 540
aagcatggat ttgattatgc aaaggactac ttcacagact taatcactaa cgagagcatt 600
aattacttca aaatgtctaa gagaatgtat ccccataggc ccgttatgat ggtgatcagc 660
cacgctgcgc cccacggccc cgaggactca gccccacagt tttctaaact gtaccccaat 720
gcttcccaac acataactcc tagttataac tatgcaccaa atatggataa acactggatt 780
atgcagtaca caggaccaat gctgcccatc cacatggaat ttacaaacat tctacagcgc 840

aaaaggctcc agactttgat gtcagtggat gattctgtgg agaggctgta taacatgctc 900
gtggagacgg gggagctgga gaatacttac atcatttaca ccgccgacca tggttaccat 960
attgggcagt ttggactggt caaggggaaa tccatgccat atgactttga tattcgtgtg 1020
ccttttttta ttcgtggtcc aagtgtagaa ccaggatcaa tagtcccaca gatcgttctc 1080
aacattgact tggcccccac gatcctggat attgctgggc tcgacacacc tcctgatgtg 1140
gacggcaagt ctgtcctcaa acttctggac ccagaaaagc caggtaacag gtttcgaaca 1200
aacaagaagg ccaaaatttg gcgtgataca ttcctagtgg aaagaggcaa atttctacgt 1260
aagaaggaag aatccagcaa gaatatccaa cagtcaaatc acttgcccaa atatgaacgg 1320
gtcaaagaac tatgccagca ggccaggtac cagacagcct gtgaacaacc ggggcagaag 1380
tggcaatgca ttgaggatac atctggcaag cttcgaattc acaagtgtaa aggacccagt 1440
gacctgctca cagtccggca gagcacgcgg aacctctacg ctcgcggctt ccatgacaaa 1500
gacaaagagt gcagttgtag ggagtctggt taccgtgcca gcagaagcca aagaaagagt 1560
caacggcaat tcttgagaaa ccaggggact ccaaagtaca agcccagatt tgtccatact 1620
cggcagacac gttccttgtc cgtcgaattt gaaggtgaaa tatatgacat aaatctggaa 1680
gaagaagaag aattgcaagt gttgcaacca agaaacattg ctaagcgtca tgatgaaggc 1740
cacaaggggc caagagatct ccaggcttcc agtggtggca acaggggcag gatgctggca 1800
gatagcagca acgccgtggg cccacctacc actgtccgag tgacacacaa gtgttttatt 1860
cttcccaatg actctatcca ttgtgagaga gaactgtacc aatcggccag agcgtggaag 1920
gaccataagg catacattga caaagagatt gaagctctgc aagataaaat taagaattta 1980
agagaagtga gaggacatct gaagagaagg aagcctgagg aatgtagctg cagtaaacaa 2040
agctattaca ataaagagaa aggtgtaaaa aagcaagaga aattaaagag ccatcttcac 2100
ccattcaagg aggctgctca ggaagtagat agcaaactgc aacttttcaa ggagaacaac 2160
cgtaggagga agaaggagag gaaggagaag agacggcaga ggaaggggga agagtgcagc 2220
ctgcctggcc tcacttgctt cacgcatgac aacaaccact ggcagacagc cccgttctgg 2280
aacctgggat ctttctgtgc ttgcacgagt tctaacaata acacctactg gtgtttgcgt 2340
acagttaatg agacgcataa ttttcttttc tgtgagtttg ctactggctt tttggagtat 2400
tttgatatga atacagatcc ttatcagctc acaaatacag tgcacacggt agaacgaggc 2460
attttgaatc agctacacgt acaactaatg gagctcagaa gctgtcaagg atataagcag 2520
tgcaacccaa gacctaagaa tcttgatgtt ggaaataaag atggaggaag ctatgaccta 2580
cacagaggac agttatggga tggatgggaa ggt 2613

<210> 3

<211> 39

<212> DNA

<213> Artificial Sequence

<220>

<223> Primer

<400> 3

tggaccaaat acaatgaagt attcttgctg tgctctggt 39

<210> 4

<211> 31

<212> DNA

<213> Artificial Sequence

<220>

<223> Primer

<400> 4

ggcaaggtca aatgaggtgt tttcccaacc t 31

<210> 5

<211> 20

<212> DNA

<213> Artificial Sequence

<220>

<223> Primer

<400> 5

gagagaggcg aatggaacga 20

<210> 6

<211> 19

<212> DNA

<213> Artificial Sequence

<220>

<223> Primer

<400> 6

cgcaccaggg agctgatct 19